# EUROPEAN PATENT SPECIFICATION

(11) **EP 0 616 797 B1**
(45) Date of publication and mention of the grant of the patent: **29.01.1997**
(21) Application number: 94104586.6
(22) Date of filing: 23.03.1994
(51) Int. Cl.: A61F 5/455, A61B 17/30

(54) **Insertion tube for a vaginal cup**
Einführröhrchen für ein Scheidenpessar
Tube d'insertion pour une couplelle vaginale

(30) Priority: 24.03.1993 DK 338/93
(43) Date of publication of application: 28.09.1994
(73) Proprietor: Geertsen, Tonni Judith, DK-8550 Ryomgaard (DK)
(72) Inventor: Geertsen, Tonni Judith, DK-8550 Ryomgaard (DK)

(56) References cited:
- WO-A-89/07919
- FR-A- 2 236 520
- US-A- 2 351 836
- US-A- 3 404 682
- US-A- 4 361 150

## Description

The present invention relates to an insertion tube for a vaginal cup for retaining menstrual liquids and comprising an elastic, flexible, substantially cylindrical, cup-shaped container with a mouth which is intended to turn upwards in the vagina.

Vaginal cups for retaining menstrual liquids have been known since the 30's. Attempts to market vaginal cups in Denmark were made in the early 70's but they were never very successful despite the advantages associated with the vaginal cup compared to the use of tampons or towels. The vaginal cup only has to be changed once or twice every 24 hours. Furthermore, when placed correctly a vaginal cup will not give rise to embarrassing smells as no oxygen will get in contact with the menstrual liquid. As the vaginal cup itself is well known, it will not be described in further detail.

A tool for inserting vaginal cups has previously been disclosed. Thus, a tool similar to forceps is known from U.S. patent No. 3 404 682 which has made it possible to hold the cup in a folded and partially compressed condition until inserted in the vagina. However, this tool is relatively complicated and has never become widespread for vaginal cups used for menstrual protection. The tool is relatively voluminous and it has been difficult for women to become familiar with using the tool.

In US-A-2 351 836 an insertion tube according to the preamble of claim 1 is disclosed.

It is the object of the present invention to disclose an insertion tube which is of a simple design and easy to use. This is achieved by the present invention as defined in claim 1.

When using a tubular insertion tube, the vaginal cup may in principle be inserted in a manner analogous to the insertion of a tampon with the exception, however, that a vaginal cup is not to be inserted as far as a tampon.

The slidable outer tube only has to have a diameter so that it fits on the outside of the inner tube. In a very simple punching operation, the inner tube, which is preferably manufactured from cardboard, may be provided with incisions extending from one end of the tube so that between the incisions jaws are formed which are connected to the remaining part of the inner tube in such a manner that they may be turned or swung in order to hold a vaginal cup folded or compressed during insertion. In a simple manner the two jaws are held together by sliding the outer tube forward over the jaws.

The vaginal cup is easily placed in the inner tube when the jaws are moved away from each other. The vaginal cup is placed in its unfolded condition between the jaws. Thereafter, the user may with an index finger fold/compress the vaginal cup through the space which an incision has created between the two jaws. At the same time, the thumb and middle finger press the jaws together when the outer tube is moved up over the jaws in order to prepare a vaginal cup for insertion.

The insertion tube preferably has two jaws provided between two V-shaped incisions, at least one of the incisions having sufficient size that the user's index finger may compress the cup at the incision.

With an insertion tube of this kind the user may easily secure that, due to its elastic flexibility, the vaginal cup has opened itself completely and is fitting tightly to the vaginal wall. This is done when the cup is in position by rotating the insertion tube a bit before pulling it out. This will ensure that the vaginal cup has unfolded itself. Outwardly oriented sealing members along the rim of the cup will provide safe contact with the vaginal wall. These sealing members will also increase the elastic unfolding force of the cup causing the jaws to be removed from each other when the cup has been positioned.

According to a preferred embodiment the insertion tube is characterized in that the inner tube has two jaws formed by providing two incisions which extend at least in the longitudinal direction of said inner tube and formed under a mutual angle of less than 180° towards the longitudinal axis of the inner tube, that said two jaws are formed coherently between said two incisions at the side where said angle is greater than 180° towards the longitudinal axis of the inner tube, and that on the other side of said incisions a cylindrical surface is provided which is designed to be able to be inserted between said two jaws as it is provided with lines of weakening for forming folding lines in order to form a sharp ridge when the vaginal cup is folded between the jaws. The inner tube is formed from one item consisting of two shell halves connected to each other by a hinge connection at the end of said inner tube which is distant from said jaws. In this embodiment the inner tube may in a simple manner be produced from plastic through die casting. It should be noted that the cylindrical surface which forms the sharp ridge when the vaginal cup is folded may be excluded as the vaginal cup may be folded by the user's finger such as is the case in the embodiment explained above in which the user's index finger compresses the cup at an incision.

The invention will now be explained in further detail with reference to the attached diagrammatic drawing, in which
- fig. 1: shows a perspective view of a vaginal cup,
- fig. 2: shows a partially sectional view of an insertion tube, the vaginal cup being partly inserted in an unfolded condition,
- fig. 3: shows schematically how the insertion tube is prepared before insertion of the vaginal cup,
- fig. 4: illustrates a sectional view through an insertion tube ready for use,
- fig. 5: shows a view of the insertion tube in a condition corresponding to fig. 2,
- fig. 6: shows a side view of a material for an inner tube according to a preferred embodiment of the insertion tube, and
- fig. 7: shows a view seen along the arrows VII-VII in fig. 6.

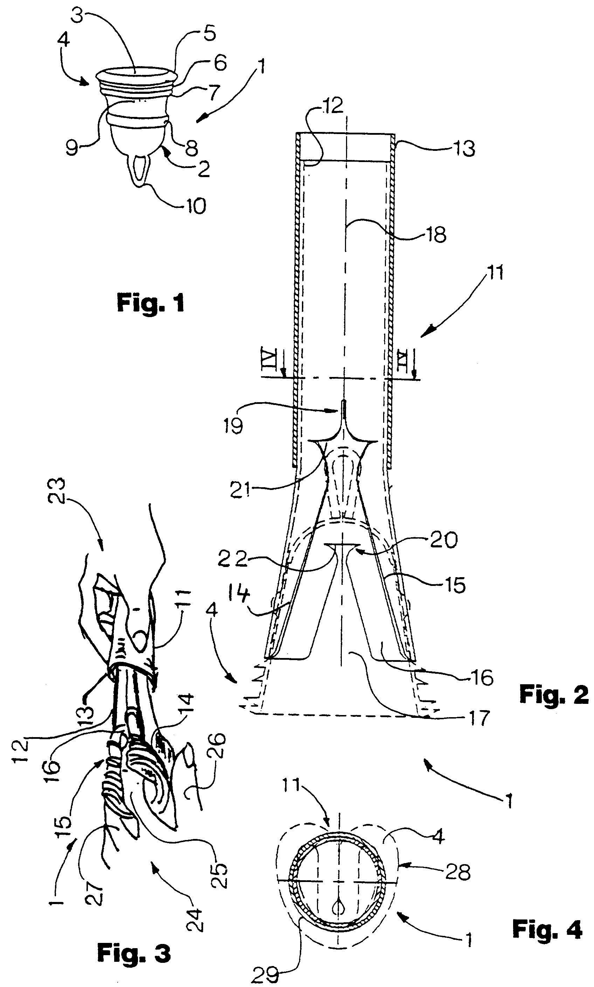

A vaginal cup 1 known in itself is shown in fig. 1. The vaginal cup is intended for retaining menstrual liquids and comprises an elastic, flexible, cup-shaped part 2 with a mouth 3 which is intended, when in use, to turn upward in the vagina. Along the mouth 3 the vaginal cup 1 is provided with sealing members 4 in the form of a number of outwardly oriented flexible ribs 5, 6, 7 intended to be in a sealing contact with the vaginal wall.

A projecting bead 8 positioned at a distance from the sealing members 4 ensures that the vaginal cup 1 will not tilt around the sealing members 4. The vaginal cup is provided with a number of ventilation openings 9 positioned immediately under the sealing members 4. When in use, the ventilation openings 9 will make it easier to position and hold the cup 1 with a weak vacuum effect. This vacuum effect is obtained, upon positioning the cup, by pulling outward very gently in a loop 10 which is positioned in the bottom of the cup 1 and which will be positioned in the vaginal orifice. The ventilation openings 9 regulate the vacuum holding the cup 1 in position. After very few minutes the vaginal cup will be so much softened by the body heat that the user no longer feels it. The vaginal cup may be manufactured from natural rubber but it is also possible to produce the cup from other materials such as Kraton, registered trademark of Shell.

Fig. 2 shows an insertion tube 11 according to the invention. The insertion tube 11 comprises an inner tube 12 and an outer tube 13 which is placed slidably on the outside of the inner tube 12. At one of its ends the inner tube 12 has two jaws 14, 15 which are capable of being moved away from each other. Alternatively, the tube may be produced with a different number of jaws than two. The jaws 14 and 15 only have to be able to contain the vaginal cup 1 when moved away from each other in the position shown in which the outer tube 13 is moved away from the jaws 14, 15.

The jaws 14, 15 are formed by providing two incisions 16, 17 opposite each other on either side of a longitudinal axis 18 through the insertion tube 11. The incision 17 has a smaller extension than the incision 16 which turns upward in the plane of the figure. The axial extension of the incisions 16, 17 corresponds approximately to the height of the vaginal cup 1. The incisions 16, 17 are provided with different depth and sizes in order to secure sufficient material stiffness and at the same time sufficient elasticity to permit easy location of the unfolded cup 1 between the jaws 14, 15.

The incisions 16 and 17 in the embodiment shown are substantially V-shaped. Immediately adjacent its bottom 19, 20, respectively, each of the incisions 16, 17 has an extension 21, 22, respectively, in the circumferential direction. These extended parts 21, 22 of the incisions permit secure swinging of the jaws 14, 15 of the inner tube 12 without any risk that the inner tube will break in the swinging point.

The inner tube and outer tube 12, 13 of the insertion tube 11 are preferably manufactured from cardboard, but they may alternatively be manufactured from plastics. It is also possible to produce the two tubes from different materials.

Fig. 3 illustrates diagrammatically how the insertion tube 11 is prepared. The user holds with one hand 23 around the withdrawn outer tube 13, which permits the jaws 14, 15 to be pulled away from each other. With the index finger 25 of the other hand 24 the user folds/compresses the cup 1 through the incision 16 so that the cup will rest folded between the jaws 14, 15 and have a smaller diameter during insertion. The user's thumb 26 and middle finger 27 press against the sides of the jaws 14, 15 and at the same time the outer tube 13 is slided forward over the inner tube 12 so that the jaws 14, 15 are retained around the folded vaginal cup 1, which is now ready for use.

The condition in which the vaginal cup 1 and the insertion tube 11 are ready for use is illustrated diagrammatically in fig. 4. It is seen that the vaginal cup has a radial extension 28 which is slightly larger than the radial outer circumference 29 of the outer tube 13. This radial extension 28 is due to the fact that the sealing members 4, as also appears from fig. 2, are placed outside the jaws 14, 15.

After preparation the insertion tube is used in the same way as with a tampon, however, the vaginal cup is not to be inserted as far into the vagina because the loop 10, as explained previously, must be visible from the outside. When the vaginal cup 1 is positioned correctly, the outer tube 13 is withdrawn. Then the insertion tube is turned slightly simultaneously with pulling slightly outwards in the loop 10. In this manner the user makes sure that the cup has opened and is fitting completely tightly to the vaginal wall via the sealing members 4.

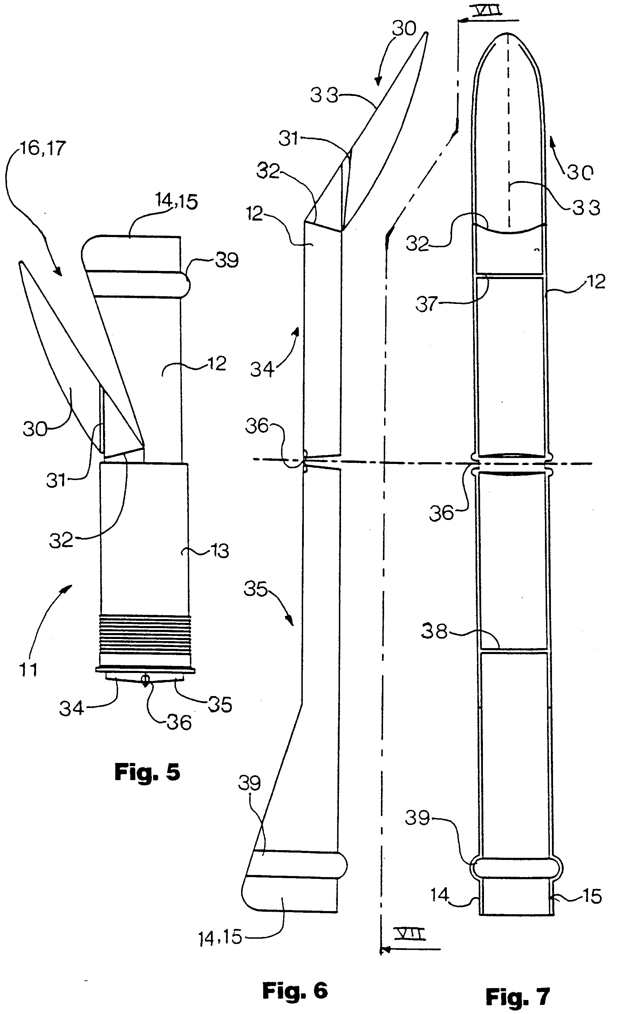

A preferred embodiment of the invention is illustrated in figs. 5 to 7. Corresponding or identical parts have received the same reference numerals and therefore will not be explained in further detail.

Fig. 5 shows an insertion tube ready for receiving a vaginal cup 1. The inner tube 12 is formed from one item such as shown in figs. 6 and 7. The jaws 14, 15 in the inner tube are formed coherently at one side of two incisions 16, 17 which are formed under a mutual angle of less than 180°. On the other side of the two incisions, at the smallest angle, a cylindrical surface 30 is provided. The cylindrical surface 30 is provided with lines of weakening 31, 32, 33 which permit the formation of a sharp ridge along the line of weakening 33 when the cylindrical surface 30 is introduced into the space between the jaws 14, 15 for folding the vaginal cup. The line of weakening at the ridge 33 which, in an "open" condition as shown in fig. 5, turn outwards will break under compression and form a sharp ridge directed towards the space between the two jaws 14, 15. After folding the vaginal cup the use of the insertion tube shown in fig. 5 will take place in the same way as explained above.

The inner tube 12 will, as shown in figs. 6 and 7, be produced from a single item consisting of two shell halves 34, 35. The two shell halves are mutually connected through a moulded hinge connection 36. When folding the two shell halves around the hinge connection 36 positioned around the end most distant from the jaws 14, 15 (see fig. 5), a cylindrical tube is formed. In order to obtain sufficient stiffness in the two shell halves 34, 36, reinforcement ribs 37, 38 are formed inside either of the shell halves 34, 35. In the end where the jaws 14, 15 are provided, a ring-shaped track 39 is formed. Said track serves to receive the bead 8 of the vaginal cup 1 so that the vaginal cup will be fixed in a particularly secure manner inside the insertion tube during insertion.

The inner tube 12 shown in figs. 6 and 7 will preferably be manufactured from a plastic material.

## Claims

1. An insertion tube (11) for a vaginal cup (4) for retaining menstrual liquids and comprising an elastic, flexible, substantially cylindrical, cup-shaped container with a mouth which is intended to turn upwards in the vagina said insertion tube comprising an outer tube (13) and two jaws (14, 15), which are capable of being moved away from each other and in which said container (4) is placed in a folded state during insertion, **characterized** in that said insertion tube further comprises an inner tube (12) having at one of its ends said jaws (14, 15), which are formed by providing two incisions (16, 17) opposite each other which extend at least in the longitudinal direction of said inner tube (12) and that said inner tube (12) is placed slidably in said outer tube (13) in order to hold said jaws (14, 15) together during insertion.

2. An insertion tube according to claim 1, **characterized** in that said two incisions (16, 17) are substantially V-shaped with a tapering away from said one end of said inner tube (12), that either incision (16, 17) has a part adjacent its bottom and extending in the circumferential direction of said inner tube, and that at the bottom of the V said incisions have an extension extending in the circumferential direction.

3. An insertion tube according to claim 2, **characterized** in that one incision (16) is considerably larger than the other incision (17).

4. An insertion tube according to any one of claims 2 to 3, **characterized** in that said two incisions have, in the longitudinal direction of the tube, an extension corresponding substantially to the length of the container.

5. An insertion tube according to claim 1, **characterized** in that said two incisions (16, 17) are formed under a mutual angle of less than 180° towards the longitudinal axis of the inner tube, that said two jaws (14, 15) are formed coherently between said two incisions (16, 17) at the side where said angle is greater than 180° towards the longitudinal axis of the inner tube, and that on the other side of said incisions (16, 17) a cylindrical surface is provided which is insertable between said two jaws (14, 15) as it is provided with lines of weakening for forming folding lines in order to form a sharp ridge when folding the vaginal cup between the jaws.

6. An insertion tube according to claim 5, **characterized** in that said inner tube (12) is formed from one item consisting of two shell halves (34, 35) connected to each other by a hinge connection (36) at the end of said inner tube (12) which is distant from said jaws (14, 15).

7. An insertion tube according to any of the preceding claims, **characterized** in that said insertion tube has a length between 100 and 150 mm, preferably between 115 and 125 mm, and an inside diameter between 19 and 27 mm, preferably between 20 and 22 mm, and that said inner tube (12) has an inside diameter between 18 and 26 mm, preferably between 19 and 21 mm for a cup with an unfolded diameter between 38 and 42 mm.

8. An insertion tube according to any of the preceding claims, **characterized** in that it is manufactured from cardboard.

9. An insertion tube according to any one of claims 1-7, **characterized** in that it is manufactured from plastic.

## Patentansprüche

1. Einführungsrohr (11) für ein Vaginalgefäß (4) zum Auffangen von menstrualen Flüssigkeiten, das einen elastischen, flexiblen, in der Hauptsache zylindrischen, glockenförmigen Behälter mit einer Öffnung umfaßt, die in der Vagina nach zeigen soll, wobei besagtes Einführungsrohr ein äußeres Rohr (13) und zwei Greifklauen (14,15) umfaßt, die von einander fortbewegt werden können und in denen der besagte Behälter (4) während des Einsetzens in gefaltenem Zustand angebracht wird, dadurch gekennzeichnet, daß besagtes Einführungsrohr außerdem ein inneres Rohr (12) mit den an einem der Enden des Rohres angebrachten besagten Greifklauen (14,15) umfaßt, die durch zwei einander gegenüber liegende Einschnitte (16,17) gebildet werden, die sich mindestens in der Längsrichtung des besagten inneren Rohres (12) erstrecken, und daß das besagte innere Rohr (12) in dem besagten äußeren Rohr (13) gleiten kann, um die besagten Greifklauen (14,15) während der Einführung zusammen zu halten.

2. Einführungsrohr nach Anspruch 1, dadurch gekennzeichnet, daß die beiden besagten Einschnitte (16,17) hauptsächlich v-förmig sind, wobei entfernt vom besagten Ende des besagten inneren Rohres (12) ein Verstärkungsring vorhanden ist, daß jeder Einschnitt am unteren Ende (16,17) einen anliegenden Teil hat, der sich kreisförmig um das besagte innere Rohr erstreckt, und daß sich am unteren Ende des besagten v-förmigen Einschnitts eine Ausbuchtung befindet, die sich in kreisförmiger Richtung erstreckt.

3. Einführungsrohr nach Anspruch 2, dadurch gekennzeichnet, daß der eine Einschnitt (16) beträchtlich länger ist als der andere Einschnitt (17).

4. Einführungsrohr nach einem der Ansprüche 2 bis 3 dadurch gekennzeichnet, daß die besagten beiden Einschnitte in der Längsrichtung des Rohres eine Ausdehnung haben, die im wesentlichen der Länge des Behälters entspricht.

5. Einführungsrohr nach Anspruch 1 dadurch gekennzeichnet, daß die besagten beiden Einschnitte (16,17) in einem wechselseitigen Winkel von weniger als 180° gegenüber der Längsachse des inneren Rohres ausgebildet sind, daß die besagten beiden Greifklauen (14,15) an der Seite, an der der Winkel gegenüber der Längsachse des inneren Rohres größer als 180° ist, zusammenhängend zwischen den Einschnitten (16,17) ausgebildet sind und daß auf der anderen Seite der Einschnitte eine zylindrische Oberfläche vorgesehen ist, die, da sie zum Ausbilden einer schaffen Kante, wenn das Vaginalgefäß zwischen den Greifklauen gefaltet wird, mit Prägelinien versehen ist, zwischen beiden Greifklauen eingesetzt werden kann.

6. Einführungsrohr nach Anspruch 5, dadurch gekennzeichnet, daß das besagte innere Rohr 12 aus einem Stück gebildet wird, das aus zwei schalenförmigen Hälften (34,35) besteht, die miteinander durch eine Gelenkverbindung (36) an dem Ende des besagten inneren Rohres (12), das von den besagten Greifklauen (14,15) entfernt ist.

7. Einführungsrohr nach einem der vorausgegangenen Ansprüche, dadurch gekennzeichnet, daß für ein Gefäß mit einem entfalteten Durchmesser von zwischen 38 und 42 mm das besagte Einführungsrohr eine Länge von zwischen 100 und 150 mm, bevorzugt zwischen 115 und 125 mm, und einen inneren Durchmesser von zwischen 19 und 27 mm, bevorzugt zwischen 20 und 22 mm, und daß das besagte innere Rohr (12) einen inneren Durchmesser von zwischen 18 und 26 mm, bevorzugt zwischen 19 und 21 mm, hat.

8. Einführungsrohr nach einem der vorausgegangenen Ansprüche, dadurch gekennzeichnet, daß es aus Karton hergestellt wird.

9. Einführungsrohr nach einem der vorausgegangenen Ansprüche, dadurch gekennzeichnet, daß es aus Kunststoff hergestellt wird.

## Revendications

1. Un tube d'insertion (11) pour une coupe vaginale (4) destinée à la rétention des liquides menstruels et comprenant un conteneur souple, flexible, fondamentalement cylindrique et en forme de coupe, muni d'une ouverture destinée à se retourner vers le haut, à l'intérieur du vagin. Le dit-tube d'insertion comprend un tube externe (13) et deux mâchoires (14) et (15) pouvant s'écarter l'une de l'autre et dans lesquelles le dit-conteneur (4) est placé, en position pliée, au cours de l'insertion. Le dit-tube d'insertion est caractérisé en ce qu'il comprend, en outre, un tube interne (12) possédant, à l'une de ses extrémités, les dites-mâchoires (14) et (15). Ces dites- mâchoires sont formées par les deux incisions (16) et (17) prévues à cet effet et sont placées l'une en face de l'autre. Ces incisions ont une extension minimum dans la direction longitudinale du dit-tube interne. Ce dit-tube interne (12) est placé de manière à coulisser dans le dit-tube externe (13) pour ainsi maintenir les dites- mâchoires (14) et (15) assemblées au cours de l'insertion.

2. Un tube d'insertion selon la déclaration 1, caractérisé en ce que les deux dites- incisions (16) et (17) sont fondamentalement en fuseau, la pointe du fuseau étant éloignée de la dite-extrémité du dit-tube interne (12). Chacune des deux incisions ont une partie adjacente à leur base et forment une extension dans la direction de la circonférence du dit-tube interne. A la base du fuseau, les dites-incisions ont une extension s'étendant dans la direction de la circonférence.

3. Un tube d'insertion selon la déclaration 2 caractérisé en ce que l'une des incisions (16) est considérablement plus grande que l'autre incision (17).

4. Un tube d'insertion selon quelqu'une des déclarations 2 à 3 caractérisé en ce que les deux dites-incisions possèdent, dans la direction longitudinale du tube, une extension correspondant fondamentalement à la longueur du conteneur.

5. Un tube d'extension selon la déclaration 1 caractérisé en ce que les dites-deux incisions (16 et 17) sont formées avec un angle mutuel n'excédant pas 180° avec l'axe longitudinal du tube interne, que les dites-deux mâchoires (14) et (15) sont formées de manière cohérente entre les dites-deux incisions (16) et (17) du côté où le dit-angle dépasse 180° avec l'axe longitudinal du tube interne et que de l'autre côté des dites-incisions (16) et (17) se trouve une surface cylindrique pouvant être insérée entre les dites-deux mâchoires (14) et (15) puisque des lignes de fléchissement existent, permettant de former des plis pour obtenir une bordure effilée lorsque la coupe vaginale est pliée entres les mâchoires.

6. Un tube d'insertion selon la déclaration 5 caractérisé en ce que le dit-tube interne (12) est formé d'un élément consistant en deux demi-coquilles (34) et (35) reliées entre elles par un joint-charnière (36) à l'extrémité du dit-tube interne (12), distant des dites-mâchoires.

7. Un tube d'insertion selon quelqu'une des déclarations précédentes caractérisé en ce que le tube d'insertion mesure entre 100 et 150 mm de long , de préférence entre 115 et 125 mm et dont le diamètre interne mesure entre 19 et 27 mm, de préférence entre 20 et 22 mm et en ce que le diamètre de tube interne (12) mesure entre 18 et 26 mm de préférence entre 19 et 21 mm pour une coupe dont le diamètre, une fois celle-ci dépliée, mesure entre 38 et 42 mm.

8. Un tube d'insertion selon quelqu'une de ces déclarations, caractérisé en ce qu'il est fabriqué en carton.

9. Un tube d'insertion selon quelqu'une des déclarations 1 à 7, caractérisé en ce qu'il est fabriqué en matière plastique.
